# EUROPÄISCHE PATENTSCHRIFT

(11) **EP 1 335 891 B1**
(45) Veröffentlichungstag und Bekanntmachung des Hinweises auf die Patenterteilung: **13.06.2007**
(21) Anmeldenummer: 01993593.1
(22) Anmeldetag: 02.11.2001
(51) Int. Cl.: C07C 29/141, C07C 31/22

(54) **VERFAHREN ZUR HYDRIERUNG VON POLY- ODER MONOMETHYLOLALKANALEN**
METHOD FOR HYDRATING POLY- OR MONOMETHYLOL ALKANALS
PROCEDE D'HYDROGENATION DE POLYMETHYLOLALCANALS ET DE MONOMETHYLOLALCANALS

(30) Priorität: 08.11.2000 DE 10055180
(43) Veröffentlichungstag der Anmeldung: 20.08.2003
(73) Patentinhaber: BASF Aktiengesellschaft, 67056 Ludwigshafen (DE)
(72) Erfinder: DERNBACH, Matthias, 69221 Dossenheim (DE); KOCH, Michael, 67346 Speyer (DE); SCHULZ, Gerhard, 67098 Bad Dürkheim (DE); WEIGL, Hagen, 68526 Ladenburg (DE); MAAS, Steffen, 55270 Bubenheim (DE)
(86) Internationale Anmeldenummer: PCT/EP2001/012681
(87) Internationale Veröffentlichungsnummer: WO 2002/038525

(56) Entgegenhaltungen:
- WO-A-98/28253
- DE-A- 19 809 418

## Beschreibung

Die Erfindung bezeiht sich auf das Gebiet der industriellen organischen Chemie. Genauer gesagt betrifft die vorliegende Erfindung ein Verfahren zur katalytischen Hydrierung von Poly- oder Monomethylolalkanalen in Gegenwart eines Kupfer-Katalysators.

Die Kondensation von Formaldehyd mit CH-aciden höheren Alkanalen zu Methylolalkanalen, im allgemeinen Dimethylol- und Trimethylolalkanalen und Überführung der erhaltenen Verbindungen in Polyole ist ein weit verbreitetes Verfahren in der Chemie. Beispiele für derart erhaltene, wichtige Triole sind Trimethylolpropan, Trimethylolethan und Trimethylolbutan, die zur Herstellung von Lakken, Urethanen und Polyestern ein breites Verwendungsgebiet gefunden haben. Weitere wichtige Verbindungen sind Pentaerythrit, erhältlich durch Kondensation von Formaldehyd und Acetaldehyd, sowie Neopentylglykol aus iso-Butyraldehyd und Formaldehyd. Der vierwertige Alkohol Pentaerythrit wird ebenfalls häufig in der Lackindustrie eingesetzt, hat aber auch eine große Wichtigkeit bei der Herstellung von Sprengstoffen erlangt.

Die erwähnten Polyole können nach verschiedenen Verfahren hergestellt werden. Eine Methode ist das sogenannte Cannizzaro-Verfahren, das noch weiterhin unterteilt wird in das anorganische und das organische Cannizzaro-Verfahren. Beide Varianten haben den Nachteil, dass neben dem Anfall schlecht abtrennbarer Nebenprodukte ein Äquivalent Formaldehyd verloren geht.

Die Nachteile des Cannizzaro-Verfahrens werden bei dem sogenannten Hydrierverfahren vermieden, das aus der WO 98/28253 bekannt ist. Dabei bringt man Formaldehyd mit dem entsprechenden Alkanal in Gegenwart von katalytischen Mengen eines Amins zur Reaktion. Damit wird erreicht, daß die Reaktion auf der Stufe des Methylolalkanals anhält. Nach Abtrennen des Formaldehyds wird das Reaktionsgemisch, das neben dem entsprechenden Alkanal noch geringe Mengen des entsprechenden Triols enthält, einer Hydrierung unterworfen, bei der das gewünschte Polyol erhalten wird.

Die katalytische Hydrierung von in Suspensions- oder Festbettfahrweise ist seit langem bekannt. Technische Anlagen arbeiten fast ausschließlich mit Festbettreaktoren.

Als Festbettkatalysatoren werden, neben Katalysatoren vom Raney-Typ wie sie beispielsweise in der WO 99/03801 beschrieben sind, jedoch vor allem geträgerte Katalysatoren, beispielsweise Kupfer-, Nickel- oder Edelmetall-Katalysatoren verwendet.

WO 99/44974 beschreibt die Hydrierung von Carbonylverbindungen, unter anderem von Poly- oder Monomethylolalkanalen, zu Alkoholen an TiO₂ geträgerten Cu-Katalysatoren.

Aus WO 95/32171 sind für die Hydrierung von Poly- oder Monomethylolalkanalen geeignete Kupferkatalysatoren auf SiO2-haltigen Trägern bekannt.

Der Erfindung lag die Aufgabe zugrunde, ein Verfahren zur katalytischen Hydrierung von Poly- oder Monomethylolalkanalen bereitzustellen, durch das nicht nur hohe Umsätze und Selektivitäten, sondern auch eine lange Katalysatorstandzeit ermöglicht.

Es wurde gefunden, dass der Gehalt der Poly- oder Monomethylolalkanalen beziehungsweise des Poly- oder Monomethylolalkanale enthaltenden Hydrierfeeds an Metallionen der Gruppen 3 bis 14 des Periodensystems der Elemente einen deutlichen Einfluss auf den erzielten Umsatz und die Selektivität der Hydrierung an kupferhaltigen Hydrierkatalysatoren sowie auf die Standzeit der kupferhaltigen Hydrierkatalysatoren hat. Unter Hydrierfeed wird der in die Hydrierung eingefahrene, aus Poly- oder Monomethylolalkanalen bestehende oder diese enthaltende Eduktstrom verstanden.

Demgemäß wurde die Aufgabe gelöst durch ein Verfahren zur katalytischen Hydrierung eines Poly- oder Monomethylolalkanals der allgemeinen Formel (I) 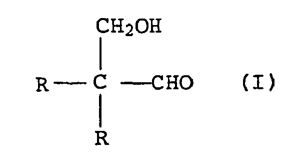 wobei R gleich oder verschieden sein kann und einen substituierten oder unsubstituierten aliphatischen Kohlenwasserstoff mit 1 bis 22 C-Atomen, eine Aryl- oder Arylalkylgruppe mit 6 bis 22 C-Atomen oder eine Methylolgruppe bedeutet, in Gegenwart eines kupferhaltigen Katalysators, dadurch gekennzeichnet, dass der das Poly- oder Monomethylolalkanal enthaltende Hydrierfeed einen Gehalt von bis 5 ppm mindestens eines Metallions der Gruppen 3 bis 14 des Periodensystems der Elemente aufweist.

Der das Poly- oder Monomethylolalkanal der allgemeinen Formel (I) enthaltende Hydrierfeed weist aus der Gruppe 3 bis 14 des Periodensystems der Elemente bevorzugt Eisen (II), Eisen (III), Chrom (III), Chrom (IV) und Nickel (II) auf. Der Gesamtgehalt des Poly- oder Monomethylolalkanale der allgemeinen Formel (I) enthaltenden Hydrierfeeds an Metallionen der Gruppen 3 bis 14 soll 5 ppm nicht übersteigen, wobei je nach Anzahl der vorhandenen Metallionen der Gehalt je Metallion 0,001 bis 5 ppm, bevorzugt 0,001 bis 2 ppm, beträgt.

Das erfindungsgemäße Verfahren kann durch Hydrierung der Reinsubstanzen oder der Mono- und Polymethylolalkane im Gemisch mit anderen Verbindungen durchgeführt werden. Da die entsprechenden Poly- oder Monomethylolalkanale beispielsweise durch Aldol-reaktion der entsprechenden aliphatischen Aldehyde mit Formaldehyd in Gegenwart eines basischen Katalysators wie in WO 98/28253 beschrieben, hergestellt werden, könnte der Reaktionsaustrag bei dieser Umsetzung direkt der Hydrierung zu den entsprechenden Polyolen zugeführt werden.

Eine vorherige Aufreinigung dieses Reaktionsaustrags ist beispielsweise aus der deutschen Patentanmeldung Nr. 199 63 445.9 bekannt, in der die Abtrennung von Formaldehyd beschrieben wird. Dabei wird insbesondere auf die Ausführungsbeispiele 2 - 4 verwiesen, soweit sie die Destillation des Aldolisierungsproduktes, d.h. des Polymethylolalkanals der allgemeinen Formel (II) betreffen. Die in den Ausführungsbeispielen genannten Reaktionsverhältnisse lassen sich analog auf Monomethylolalkanale übertragen. Eine Abtrennung von Metallionen aus dem Reaktionsaustrag ist nicht beschrieben. Zudem geht durch die Destillation Poly- oder Monomethylolalkanal verloren.

Metallionen gelangen in der Regel durch Korrosion von Anlagenteilen in der Aldolisierung in das Poly- oder Monomethylolalkanal beziehungsweise in den Poly- oder Monomethylolalkanal enthaltenden Hydrierfeed. Poly- oder Monomethylolalkanal beziehungsweise den Poly- oder Monomethylolalkanal enthaltender Hydrierfeed mit verringertem Metallionengehalt kann daher zum einen durch die Vermeidung des Eintrags von Metallioneneintrags, zum Beispiel durch die Wahl geeigneter metallfreier Werkstoffe wie Glas, Email oder hochwertigen Werkstoffen wie Titan oder hochwertigen Legierungen für Lager- und Zwischenbehälter, Rohrleitungen, Reaktoren, Destillations- und Rektifikationskolonnen erhalten werden. Da dies, wenn es technisch realisierbar ist, insbesondere für grosstechnische Anlagen mit erheblichen Kostenaufwand verbunden sein kann, ist es bevorzugt den Metallionengehalt durch Abtrennung der Metallionen aus den Poly- oder Monomethylolalkanalen und/oder den zu ihrer Herstellung erforderlichen Edukten bzw. deren Prozessströmen, wie zum Beispiel bei der Aldolreaktion zur Herstellung der Poly- oder Monomethylolalkanale den entsprechenden aliphatischen Aldehyde und Formaldehyd, zu reduzieren.

Die Abtrennung der Metallionen aus den Poly- oder Monomethylolalkanalen und/oder den zu ihrer Herstellung erforderlichen Edukten wie Formaldehyd oder aliphatischen Aldehyden kann durch Behandlung mit Absorbentien und/oder durch Komplexierung und nachfolgende Membrantrennverfahren erfolgen, bevorzugt erfolgt sie durch Behandlung mit Absorbentien.

Für die erfindungsgemäße Komplexierung mit nachfolgendem Membrantrennungsverfahren wird der Hydrierfeed mit einem hochpolymeren, löslichen Komplexierungsmittel versetzt, das die im Feed enthaltenen Metallionen komplexiert. Als Komplexierungsmittel können Polymere jeglicher Art eingesetzt werden, die zur Komplexierung geeignete funktionelle Gruppen (beispielsweise COOH, NR₂, etc) oder Heteroatome wie N oder P enthalten. So können beispielsweise Polyimine geeigneter Molmasse eingesetzt werden. Das komplexierte Polymer und der Überschuss an nicht-komplexiertem Polymer wird anschließend über eine geeignete Membran (organisch oder anorganisch) vom Hydrierfeed abgetrennt. Die Membran hält das Komplexierungsmittel samt gebundenen Metallionen zurück, der (gelöste) Hydrierfeed passiert die Membran und wird anschließend hydriert.

Bevorzugt werden als Absorbentien Aktivkohlen, saure oder basische Ionentauscher oder deren Gemische, Metalloxide oder Molekularsiebe eingesetzt, wobei Chelationentauscher besonders bevorzugt sind.

Erfindungsgemäß wurde erkannt, dass keine Zersetzung der empfindlichen Polymethylolalkanale bei Behandlung mit den Absorbentien eintritt. Dies ist überraschend, da es bekannt ist, dass Polymethylolalkanale bei höherer Temperatur sowie bei basischer oder saurer Behandlung zur Retro-Aldol-Reaktion, das heisst zur Rückreaktion ihrer Bildung, neigen.

Geeignete Aktivkohlen weisen beispielweise einer Oberfläche von 500 bis 2000 m²/g nach DIN 66 131 und einer Porosität von 0,05 bis 1,0 cm³/g nach DIN 66134 auf und werden von der Firma Merck, Darmstadt, von der Firma Chemviron Midwest Corp., Wooster, USA, unter der Handelsbezeichnung CPG LF 8 30^{®} und der Firma Lurgi AG Frankfurt, unter der Handelsbezeichnung Carboraffin P^{®} vertrieben.

Geeignete Ionentauschern sind bevorzugt Chelationentauscher wie zum Beispiel Amberlite^{®} TRL der Firma Rohm & Haas, Darmstadt, Levatit^{®} TP 207 der Firma Bayer AG, Leverkusen, in allen möglichen Formen, beispielsweise körnig oder als Gel.

Als Metalloxide können zum Beispiel alpha- oder gamma-Aluminiumoxid, Siliciumoxid, Titandioxid in der Anastas oder Rutilmodifikation, Zirkondioxid, Magnesiumoxid, Calciumoxid, Zinkoxid oder Gemische von Metalloxiden wie zum Beispiel Aluminosilikate verwendet werden. Geeignetes Aluminiumoxid wird beispielsweise von der Firma Condea Chemie AG, Hamburg, unter der Handelsbezeichnung Pural^{®} SB vertrieben.

Geeignete Molekularsiebe sind beispielsweise Alumosilikate oder Zeolithe mit einem Porendurchmesser von grösser 3 Ä, beispielsweise Zeokat ^{®}Z 6 01-01-y Zeolith oder Zeochem^{®} Molsieb 13x13 Zeolith der Firma Uetikon AG, Uetikon, Schweiz.

Die Absorbentien können in Form von Formkörpern wie zum Beispiel Kugeln, Strängen, Tabletten, Splitt oder Pulver vorliegen.

In der Regel wird das Poly- oder Monomethylolalkanal und/oder das zu ihrer Herstellung erforderliche Edukt bei einer Temperatur zwischen dem Erstarrungspunkt und dem Siedepunkt des Poly- oder Monomethylolalkanals oder des zu ihrer Herstellung erforderlichen Edukts, dessen Metallionengehalt gesenkt werden soll, bevorzugt bei 20 bis 150°C, besonders bevorzugt bei 40 bis 100 °C, und Drukken von 0,001 bis 200 bar, bevorzugt bei 0,5 bis 10 bar, in einem Rührbehälter, bevorzugt jedoch durch Durchleiten durch ein Absorbents im Festbett, behandelt.

In einer besonders bevorzugten Ausführungsform werden die Poly- oder Monomethylolalkanale durch Aldolreaktion aus den entsprechenden aliphatischen Aldehyden und Formaldehyd in Gegenwart eines basischen Katalysators gemäß WO 98/28253, auf die hier ausdrücklich Bezug genommen wird, hergestellt. Der Reaktionaustrag der Aldolisierung gemäß WO 98/28253 wird, bevorzugt auf der noch drucklosen Seite, direkt über ein Absorbens im Festbett, besonders bevorzugt einen Chelationentauscher, geleitet und sodann der Hydrierung zu den entsprechenden Polyolen zugeführt. Bei Verwendung von Ionentauschern als Absorbens sollte innerhalb der vorstehend genannten Temperaturbereiche die Herstellerempfehlung für den optimalen Temperaturbereich des jeweiligen Ionentauschers berücksichtigt werden.

Die Verweilzeit des Poly- oder Monomethylolalkanals oder des zu ihrer Herstellung erforderlichen Edukts ist von der Affintät des Absorbens abhängig und liegt in der Regel zwischen 1 min und 24 h, bevorzugt jedoch bei 5 bis 30 min. Es ist möglich das Poly- oder Monomethylolalkanal oder das zu ihrer Herstellung erforderliche Edukt in einem Lösungsmittel, bevorzugt in Form einer 20 bis 60 Gew.-% igen Lösung, einzusetzen. Als Lösungsmittel sind beispielsweise Wasser, Alkohole wie beispielsweise Methanol und Ethanol, oder 0, 1 bis 99 % ige Alkohol-Wassergemische geeignet. Bevorzugt wird die Behandlung mit dem Absorbents in Wasser oder einem 0, 1 bis 99 % igen Alkohol-Wassergemisch durchgeführt.

Das Absorbens kann regeneriert werden, z.B. abhängig vom Absorbens durch Spülen mit Wasser, Laugen oder Säuren und anschließendes Waschen mit Wasser. Saure oder stark saure Ionentauscher werden bevorzugt mit wässriger Salzsäure, Schwefelsäure, Ameisensäure oder Essigsäure regeneriert, während basische oder stark basische Ionentauscher bevorzugt mit wässriger Natronlauge, Kalilauge oder Ca(OH)₂ regeneriert werden können.

Die Vermeidung des Eintrags von Metallioneneintrags wird besonders bevorzugt mit der Behandlung der Poly- oder Monomethylolalkanale oder der zu ihrer Herstellung erforderlichen Edukte mit einem Absorbens kombiniert.

Für die erfindungsgemäße Hydrierung werden kupferhaltige Katalysatoren eingesetzt, bevorzugt solche die ausgewählt sind aus Raney-Kupfer-Katalysatoren und geträgerten Kupferkatalysatoren.

Geeignete Raney-Nickel-Katalysatoren sind beispielsweise die Raney-Nickel-katalysatoren in Form von Nuggets, die aus der WO 99/03801 bekannt sind, auf die hier ausdrücklich Bezug genommen wird. Diese Katalysatoren weisen eine Korngröße der Nuggets von 2 bis 7 mm, eine Kupfergehalt von 40 bis 90 Gew.-%, eine Oberfläche nach Langmuir von 5 bis 50 m²/g, eine Kupferoberfläche von 0,5 bis 7 m²/g, ein Hg-Porenvolumen von 0,01 bis 0,12 ml/g und einen mittleren Porendurchmesser von 50 bis 300 nm auf.

Für die Verwendung im erfindungsgemäßen Verfahren geeignete geträgerte Kupferkatalysatoren sind beispielsweise die aus der WO 95/32 171, auf die hier ausdrücklich Bezug genommen wird, bekannten auf SiO₂ geträgerten Kupferkatalysatoren, die 5 bis 50 Gew.-% Kupfer, berechnet als CuO, vorzugsweise 70 bis 95 Gew.-% Silicium, berechnet als SiO₂, sowie gegebenenfalls eines oder mehrere der Elemente Magnesium, Barium, Zink oder Chrom enthalten, und durch Tränkung eines porösen Siliciumdioxid-Trägermaterials mit einer überstehenden wässrigen Lösung einer thermisch leicht zersetzbaren Kupferverbindung, anschließende Trocknung und Calcinierung bei 200 bis 400°C hergestellt werden.

Besonders bevorzugt wird die erfindungsgemäße Hydrierung in Gegenwart des aus der WO 99/44974, auf die hier ausdrücklich Bezug genommen wird, bekannten Katalysators, der einen anorganischen Träger, der TiO₂ enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfaßt und dessen spezifische Kupferoberfläche maximal 10 m²/g beträgt, durchgeführt. Diese Katalysatoren weisen bevorzugt als Träger TiO₂ oder eine Mischung aus TiO₂ und Al₂O₃ oder eine Mischung aus TiO₂ und ZrO₂ oder eine Mischung aus TiO₂, Al₂O₃ und ZrO₂ auf, besonders bevorzugt wird TiO₂ verwendet. Bei der Herstellung dieses Katalysators gemäß WO 99/ 44974 kann metallisches Cu-Pulver als weiteres Additiv während der Tablettierung zugesetzt werden, dass die Kupferoberfläche maximal 10 m²/g beträgt.

Bevorzugt wird die Hydrierung im Festbett durchgeführt. Die Ausführungsform als eine kontinuierliche oder diskontinuierliche Suspensionshydrierung sowie die Ausführungsform als Wirbelbettreaktion mit auf- und abwirbelnder Bewegung befindlichem Katalysatormaterial ist jedoch ebenfalls möglich. Die Hydrierung kann in der Gasphase oder in der Flüssigphase durchgeführt werden. Vorzugsweise wird die Hydrierung in flüssiger Phase durchgeführt, beispielsweise in Riesel- oder Sumpffahrweise.

Bei Arbeiten in Rieselfahrweise läßt man das flüssige, das zu hydrierende Poly- oder Monmethylolalakanal enthaltende Edukt in dem Reaktor, der unter Wasserstoffdruck steht, über das in diesem angeordnete Katalysatorbett rieseln, wobei sich auf dem Katalysator ein dünner Flüssigkeitsfilm ausbildet. Dagegen wird beim Arbeiten in Sumpffahrweise Wasserstoffgas in den mit der flüssigen Reaktionsmischung gefluteten Reaktor eingeleitet, wobei der Wasserstoff das Katalysatorbett in aufsteigenden Gasperlen passiert.

In einer Ausführungsform wird die zu hydrierende Lösung im geraden Durchgang über die Katalysatorschüttung gepumpt. In einer anderen Ausführungsform des erfindungsgemäßen Verfahrens wird ein Teil des Produkts nach Durchgang durch den Reaktor als Produktstrom kontinuierlich abgezogen und ggf. durch einen zweiten Reaktor, wie oben definiert, geleitet. Der andere Teil des Produkts wird zusammen mit frischem, die Poly- oder Monmethylolalakanal enthaltendem Edukt dem Reaktor erneut zugeführt. Diese Verfahrensweise wird im folgenden als Kreislauffahrweise bezeichnet.

Wird als Ausführungsform des erfindungsgemäßen Verfahrens die Rieselfahrweise gewählt, ist hierbei die Kreislauffahrweise bevorzugt. Weiter bevorzugt wird in Kreislauffahrweise unter Verwendung eines Haupt- und Nachreaktors gearbeitet, wobei besonders bevorzugt der Hauptreaktor in Kreislauffahrweise und der Nachreaktor im geraden Durchgang betrieben wird.

Das erfindungsgemäße Verfahren eignet sich zur Hydrierung von Poly- und Monomethylolverbindungen. Beispiele sind Dimethylolethanal, Trimethylolethanal (Pentaerythrital), 2-Methylolpropanal, 2,2-Dimethylolpropanal (DMP), 2-Methylolbutanal, 2,2-Dimethylolbutanal, Hydroxypivalinaldehyd. Besonders bevorzugt sind Hydroxypivalinaldehyd (HPA), Trimethylolethanal (Pentaerythrital), 2,2-Dimethylolpropanal (DMP) und Dimethylolbutanal (DMB).

Das zu hydrierende Poly- oder Monomethylolalkanal kann dem Hydrierungsreaktor allein oder als Gemisch mit dem Produkt der Hydrierungsreaktion zugeführt werden, wobei dies in unverdünnter Form oder unter Verwendung von zusätzlichem Lösungsmittel geschehen kann. Als zusätzliches Lösungsmittel eigenen sich insbesondere Wasser, Alkohole wie Methanol, Ethanol und der Alkohol, der unter den Reaktionsbedingungen entsteht. Bevorzugte Lösungsmittel sind Wasser, THF, NMP, sowie Ether, wie z.B. Dimethyl-, Diethylether, MTBE, besonders bevorzugt ist Wasser.

Die Hydrierung sowohl in Sumpf- als auch in Rieselfahrweise, wobei jeweils bevorzugt in Kreislauffahrweise gearbeitet wird, führt man im allgemeinen bei einer Temperatur von 20 bis 250°C, bevorzugt bei 70 bis 200°C, besonders bevorzugt bei 80 bis 150°C und einem Druck von 1 bis 350 bar, bevorzugt 10 bis 200 bar, besonders bevorzugt 20 bis 100 bar durch.

Mit dem erfindungsgemäßen Verfahren werden hohe Umsätze und Selektivitäten erzielt, und die Katalysatoren weisen eine hohe Standzeit auf. Die Erfindung wird in den nachstehenden Beispielen näher erläutert.

### Beispiele

### Herstellung Dimethylolbutyraldehyd

Die Erfindung wird nun in den nachfolgenden Beispielen erläutert. In diesen Beispielen wurde ein Aldolisierungsgemisch verwendet, das wie folgt dargestellt worden war:

Eine Apparatur bestehend aus zwei heizbaren, durch Überlaufrohre miteinander verbundenen Rührkessen mit einem Fassungsvermögen von insgesamt 72 l wurde mit frischer, wässriger Formaldehydlösung (4300 g/h einer 40%igen wässrigen Lösung bzw. n-Butyraldehyd (1800 g/h) und mit frischem Trimethylamin als Katalysator (130 g/h) in Form einer 45%igen wässrigen Lösung kontinuierlich beschickt. Die Reaktoren wurden auf 40°C temperiert. Es kann dabei ein methanolarmes Formaldehyd verwendet werden , wie dies in der deutschen Anmeldung DE 199 63 438.6 mit dem Titel " Verfahren zur Herstellung von Polyalkoholen mit Methanol-armen Formaldehyd" (Anmelderin: BASF AG) vom 28.12.1999 beschrieben ist.

Der Austrag wurde direkt über einen Fallfilmverdampfer mit aufgesetzter Kolonne (11 bar Heizdampf) geleitet und dort bei normalem Druck destillativ in ein leichsiedendes Kopfprodukt, im wesentlichen enthaltend Butyraldehyd, Ethylacrolein, Formaldehyd, Wasser und Trimethylamin und ein hochsiedendes Sumpfprodukt aufgetrennt.

Das Kopfprodukt wurde kontinuierlich kondensiert und in die oben beschriebenen Reaktoren zurückgeführt.

Das hochsiedende Sumpfprodukt aus dem Verdampfer (ca. 33, 5 kg/h) wurde kontinuierlich mit frischem Trimethylamin-Katalysator (50g/h in Form einer 45%igen wässrigen Lösung) versetzt und in einen beheizbaren, mit Füllkörpern versehenen Rohrreaktor mit einem Leervolumen von 12 l geführt. Der Reaktor war dabei auf 40°C temperiert.

Der Austrag des Nachreaktors wurde kontinuierlich in den oberen Teil einer weitern Destillatiosneinrichtung, der Formaldehydabtrennung, (11 bar Heizdampf) gegeben und dort destillativ in ein leicht siedendes Kopfprodukt, im wesentlichen enthaltend Ethylacrolein, Formaldehyd, Wasser und Trimethylamin, und ein hochsiedendes Sumpfprodukt aufgetrennt. Das leichtsiedende Kopfprodukt (27 kg/h) wurde kontinuierlich kondensiert und in den ersten Rührkessel zurückgeleitet, wohingegen das hochsiedende Sumpfprodukt gesammelt wurde.

Das so erhaltenen Sumpfprodukt enthielt neben Wasser im wesentlichen Dimethylolbutyraldehyd, Formaldehyd und Spuren Monomethylobutyraldehyd.

Die Abtrennung des Formaldehyds aus dem Sumpfprodukt erfolgt nach Beispiel 4 der deutschen Anmeldung DE 199 63 445.9 mit dem Titel "Verfahren zur Abtrennung von Formaldehyd aus polyolhaltigen Reaktionsgemischen unter Zusatz von Lösungsmitteln"vom 28.12.1999 (Anmelderin : BASF AG). Es wurde ein 70%iger wässriger Austrag erhalten, der 24 Gew.-% Dimthylolbutyralaldehyd (DMB) enthielt.

### Bestimmung des Metallionengehalts

0,5 bis 1 g einer Probe eines Poly- oder Monomethylolalkanal enhaltenden Hydrierfeeds wurden einem Säureaufschlussverfahren unterworfen, bei dem man die Probe zunächst mit 0,2 ml einer wässrigen Na₂SO₄-lösung (200g/l Na₂SO₄), sodann mit 8 ml Schwefelsäure (1,84 g/ml) und anschliessend mit 3 ml Salpetersäure (1,41 g/ml) versetzt und dann zum Sieden erhitzt. Sodann wird in der Hitze mit 10 ml eines Gemisches aus Salpetersäure, Schwefelsäure und Perchlorsäure im Volumenverhältnis 2:1:1 oxidiert. Nach dem Abrauchen der überschüssigen Säuren füllt man mit verdünnter Salzsäure auf 10 ml auf. Aus dieser Meßlösung wird die Metallionenkonzentration durch Atomemissionsspektroskopie mit induktiv gekoppeltem Plasma (ICP-AES) beispielsweise mit dem ICP-Spektrometer IRIS Advantage der Firma Thermo-Jarrel Ash bestimmt.

### Beispiele 1 bis 6

Eine nach obiger Vorschrift hergestellte Cr- und Ni-Ionen enthaltende 70% wässrige DMB-Lösung (70°C) wurde kontinuierlich in Sumpffahrweise über ein mit verschiedenen Adsorbensmaterialien gefüllten Rohrreaktor gefahren. Der 30ml-Reaktor wurde jeweils voll mit Adsorbens befüllt, die Zulaufmenge der DMB-Lösung betrug 30 ml/h. Die Austräge wurden gesammelt und auf Cr- und Ni-Gehalt analytisch überprüft. Das Ergebnis ist in Tabelle 1 zusammengefasst.

**Tabelle 1**

| Beispiel | Adsorbens | Cr [ppm] | Ni [ppm] |
|---|---|---|---|
| Edukt | | 13 | 17 |
| 1 | 72281 Pural^{®} SB (1,5mm-Stränge) | <1 | <1 |
| 2 | γ-Al₂O₃ (3mm-Stränge) | <1 | 1 |
| 3 | Molsieb 10Å (Kugeln) | <1 | <1 |
| 4 | Zeolith (Zeokat^{®} Z6 01-01 y) | 1 | 8 |
| 5 | TiO₂ (3mm-Stränge) | <1 | 11 |
| 6 | Zeolith (Zeochem^{®} Molsieb 13x13) | 3 | <1 |

### Beispiel 7 und 8

Die Beispiele wurden analog den Beispielen 1 bis 6 durchgeführt, wobei jedoch ein 60ml-Reaktor verwendet wurde und die Zulaufmenge 15 ml/h betrug. Das Ergebnis ist in Tabelle 2 zusammengefasst.

**Tabelle 2**

| Beispiel | Adsorbens | Fe [ppm] | Cr [ppm] | Ni [ppm] |
|---|---|---|---|---|
| Edukt | | 50 | 10 | 10 |
| 7 | Chelationentauscher (Lewatit^{®} TP 207) | <1 | <1 | <1 |
| 8 | Chelationentauscher (Amberlite^{®}TRL 218) | <1 | <1 | 1 |

### Beispiel 9 bis 11

Bei Raumtemperatur einer wässrige Lösung von Dimethylolpropionaldehyd (Gehalt von 37,2% Dimethylolpropinaldehyd), die zu Beginn einen Gehalt von 21 ppm Eisenionen aufwies, mit 10 Gew% Aktivkohlen 15h gerührt. Nach Abtrennung der Aktivkohle wurde die Fe-Konzentration im Austrag bestimmt. Das Ergebnis ist in Tabelle 3 zusammengefasst

**Tabelle 3**

| Beispiel Nr.: | Aktivkohle | Konzentration Fe [ppm] |
|---|---|---|
| 9 | Merck (Pulver) | 1 |
| 10 | Carboraffin P^{®} | 0,5 |

### Vergleichsbeispiel V1

Die Dimethylolaldehyd-Hydrierung wurde in einem Festbettreaktor (Hauptreaktor, Kreislauffahrweise) an 150 ml/h Katalysator und einem weiteren nachgeschalteten 2. Festbettreaktor (Nachreaktor, gerader Durchgang) an 50 ml/h cu-TiO₂-Katalysator durchgeführt. Der Katalysator wurde analog J der DE 198 09 418 hergestellt. Er enthielt 42 Gew% CuO, 16 Gew% Cu und 46% TiO₂. Der Katalysator wurde zuvor bei 180°C mit einem Wasserstoff/Stickstoff-Gemisch aktiviert. Die Hydrierung des methylolaldehyd-haltigen Einsatzstoffes erfolgte in Rieselfahrweise bei einer Temperatur von 120°C, einem Druck von 90 bar, einem Zulauf von 100 ml/h und einer Kreislaufmenge im Hauptreaktor von 900 ml/h.

Die Analyse der wässrigen Dimethylolbutyraldehyd (DMB)-Feeds zeigte 10 ppm Ni, 10 ppm Cr und 3ppm Fe. Die Hydrierung wurde 12 Tage unter diesen Bedingungen betrieben. Das Ergebnis ist in Tabelle 4 zusammengefasst.

**Tabelle 4**

| Laufzeit [h] | DMB nach Hauptreaktor [GC-FI%] | Umsatz [%] nach Hauptreaktor [%] | Selektivität [%] |
|---|---|---|---|
| 24 h | 0,20 | 99,1 | 94,2 |
| 144 h | 0,97 | 95,8 | 93,1 |
| 288 h | 2,66 | 88,4 | 92,6 |

### Beispiel 11

Analog dem Vergleichsbeispiel V1 wurde über den gleichen Zeitraum eine kontinuierliche Hydrierung durchgeführt, wobei jedoch ein Dimethylolbutyraldehyd-Feed eingesetzt wurde, der 1 ppm Cr, 1 ppm Ni und 2 ppm Fe enthielt. Das Ergebnis ist in Tabelle 5 zusammengefasst.

**Tabelle 5**

| Laufzeit [h] | DMB nach Hauptreaktor [GC-FI%] | Umsatz [%] | Selektivität [%] |
|---|---|---|---|
| 24 | 0,42 | 98,2 | 94,4 |
| 144 | 0,47 | 98,0 | 94,6 |
| 288 | 0,55 | 97,6 | 94,5 |

Ein Vergleich des Ergebnisses des erfindungsgemäßen Beispiels mit dem dem Vergleichsbeispiel ist in der Tabelle 6 zusammengefasst.

**Tabelle 6**

| Beispiel | Metallgehalt Fe+Cr+Ni | Umsatzverlust nach 288h | Selektivitätsverlust nach 288h |
|---|---|---|---|
| 11 | 4 ppm | 0,6% | 0,1% |
| V1 | 23 ppm | 10,7% | 1,6% |

Der Vergleich zeigt, dass mit dem erfindungsgemäßen Verfahren eine deutlich höhere Standzeit des Katalysators verwirklicht werden kann.

## Patentansprüche

1. Verfahren zur katalytischen Hydrierung eines Poly- oder Monomethylolalkanals der allgemeinen Formel (I) 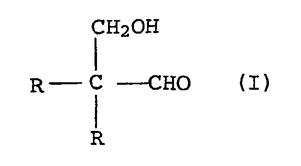 wobei R gleich oder verschieden sein kann und einen substituierten oder unsubstituierten aliphatischen Kohlenwasserstoff mit 1 bis 22 C-Atomen, eine Aryl- oder Arylalkylgruppe mit 6 bis 22 C-Atomen oder eine Methylolgruppe bedeutet, in Gegenwart eines kupferhaltigen Katalysators, **dadurch gekennzeichnet, dass** der das Poly- oder Monomethylolalkanal enthaltende Hydrierfeed einen Gesamtgehalt bis 5 ppm Metallionen der Gruppen 3 bis 14 des Periodensystems der Elemente aufweist.

2. Verfahren nach Anspruch 1, **dadurch gekennzeichnet, dass** der Gehalt je Metallion der Gruppen 3 bis 14 des Periodensystems der Elemente 0,001 bis 5 ppm beträgt.

3. Verfahren nach Anspruch 1 oder 2, **dadurch gekennzeichnet, dass** die Einstellung des Metallionengehalt des das Poly- oder Monomethylolalkanal enthaltenden Hydrierfeed durch Abtrennung der Metallionen aus den Poly- oder Monomethylolalkanalen und/oder den zu ihrer Herstellung erforderlichen Edukten durch Behandlung mit Absorbentien und/oder durch Komplexierung und nachfolgende Membrantrennverfahren und/ oder Vermeidung des Eintrag der Metallionen erfolgt.

4. Verfahren nach einem der Ansprüche 1 bis 3, **dadurch gekennzeichnet, dass** das Absorbens ausgewählt ist aus Aktivkohlen, sauren oder basischen Ionentauschern oder deren Gemischen, Metalloxiden oder Molekularsieben.

5. Verfahren nach Anspruch 4, **dadurch gekennzeichnet, dass** das Adsorbens ein Chelationentauscher ist.

6. Verfahren nach Anspruch 1, **dadurch gekennzeichnet, dass** der kupferhaltige Katalysator ausgewählt ist aus Raney-Kupfer-Katalysatoren und geträgerten Kupferkatalysatoren auf oxidischen Trägern.

7. Verfahren nach Anspruch 1, **dadurch gekennzeichnet, daß** der kupferhaltige Katalysators, einen anorganischen Träger, der TiO₂ enthält, aufweist und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfaßt, und die Kupferoberfläche maximal 10 m²/g beträgt.

8. Verfahren nach Anspruch 7, **dadurch gekennzeichnet, dass** das Trägermaterial eine Mischung aus TiO₂ und Al₂O₃ oder eine Mischung aus TiO₂ und ZrO₂ oder eine Mischung aus TiO₂ und Al₂O₃ und ZrO₂ umfaßt.

9. Verfahren nach Anspruch 7 oder 8, **dadurch gekennzeichnet, daß** das Katalysatormaterial unter Zusatz von metallischem Kupferpulver zu Tabletten verformt wird.

10. Verfahren nach einem der Ansprüche 1 bis 9, **dadurch gekennzeichnet, dass** die katalytische Hydrierung als Festbettreaktion in Rieselfahrweise oder in Sumpffahrweise durchgeführt wird.

11. Verfahren nach einem der Ansprüche 1 bis 10, **dadurch gekennzeichnet, dass** das Verfahren in Kreislauffahrweise durchgeführt wird.

## Claims

1. A process for the catalytic hydrogenation of a polymethylolalkanal or a monomethylolalkanal of the formula (I) 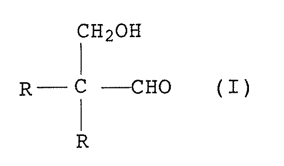 where R may be identical or different and are each a substituted or unsubstituted aliphatic hydrocarbon having from 1 to 22 carbon atoms, an aryl or arylalkyl group having from 6 to 22 carbon atoms or a methylol group, in the presence of a copper-containing catalyst, wherein the hydrogenation feed comprising the polymethylolalkanal or monomethylolalkanal has a total content of metal ions of groups 3 to 14 of the Periodic Table of the Elements of up to 5 ppm.

2. The process according to claim 1, wherein the content of metal ions of groups 3 to 14 of the Periodic Table of the Elements is from 0.001 to 5 ppm.

3. The process according to claim 1 or 2, wherein the metal ion content of the hydrogenation feed comprising the polymethylolalkanal or monomethylolalkanal is set by removal of the metal ions from the polymethylolalkanal or monomethylolalkanal and/or the starting materials required for its preparation by treating with adsorbents and/or by complexation and subsequent membrane separation processes and/or avoidance of the introduction of the metal ions.

4. The process according to any of claims 1 to 3, wherein the adsorbent is selected from among activated carbon, acid and base ion exchangers and mixtures thereof, metal oxides and molecular sieves.

5. The process according to claim 4, wherein the adsorbent is a chelating ion exchanger.

6. The process according to claim 1, wherein the copper-containing catalyst is selected from among Raney copper catalysts and supported copper catalysts on oxidic supports.

7. The process according to claim 1, wherein the copper-containing catalyst comprises an inorganic support comprising TiO₂ and, as active component, copper or a mixture of copper with at least one metal selected from the group consisting of zinc, aluminum, ceriuim, noble metals and metals of transition group VIII, and the copper surface area is not more than 10 m²/g.

8. The process according to claim 7, wherein the support material comprises a mixture of TiO₂ and Al₂O₃ or a mixture of TiO₂ and ZrO₂ or a mixture of TiO₂ and Al₂O₃ and ZrO₂.

9. The process according to claim 7 or 8, wherein metallic copper powder is added to the catalyst material during shaping to produce pellets.

10. The process according to any of claims 1 to 9, wherein the catalytic hydrogenation is carried out as a fixed-bed reaction in the downflow mode or in the upflow mode.

11. The process according to any of claims 1 to 10 carried out in the circulation mode.

## Revendications

1. Procédé d'hydrogénation catalytique d'un polyméthylolalcanal ou d'un monométhylolalcanal de formule générale (I) : 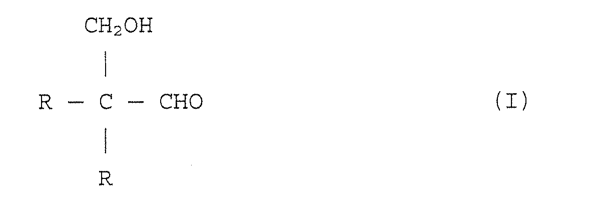 dans laquelle les R peuvent être identiques ou différents et représentent un hydrocarbure aliphatique substitué ou non substitué comportant 1 à 22 atomes de C, un groupe aryle ou arylalkyle comportant 6 à 22 atomes de C ou un groupe méthylol, en présence d'un catalyseur contenant du cuivre, **caractérisé en ce que** l'alimentation de l'hydrogénation qui contient du polyméthylolalcanal ou du monométhylolalcanal présente une teneur globale allant jusqu'à 5 ppm d'ions métalliques des groupes 3 à 14 de la classification périodique des éléments.

2. Procédé suivant la revendication 1, **caractérisé en ce que** la teneur de chaque ion métallique des groupes 3 à 14 de la classification périodique des éléments est de 0,001 à 5 ppm.

3. Procédé suivant la revendication 1 ou 2, **caractérisé en ce que** l'ajustement de la teneur en ions métalliques de l'alimentation de l'hydrogénation contenant le polyméthylolalcanal ou monométhylolalcanal est effectué par isolement des ions métalliques à partir des polyméthylolalcanals ou monométhylolalcanals et/ou des matières de départ nécessaires pour leur préparation, par traitement avec des agents absorbants et/ou par complexation et un procédé de séparation par membrane ultérieur et/ou en évitant l'introduction des ions métalliques.

4. Procédé suivant l'une des revendications 1 à 3, **caractérisé en ce que** l'agent absorbant est choisi parmi du charbon actif, des échangeurs d'ions acides ou basiques ou leurs mélanges, des oxydes métalliques ou des tamis moléculaires.

5. Procédé suivant la revendication 4, **caractérisé en ce que** l'agent absorbant est un échangeur d'ions de chélation.

6. Procédé suivant la revendication 1, **caractérisé en ce que** le catalyseur contenant du cuivre est choisi parmi des catalyseurs à cuivre de Raney et des catalyseurs à base de cuivre sur support sur des supports oxydés.

7. Procédé suivant la revendication 1, **caractérisé en ce que** le catalyseur contenant du cuivre présente un support inorganique, qui contient du TiO₂, et comporte, comme composant actif, du cuivre ou un mélange de cuivre avec au moins un des métaux choisis parmi le groupe du zinc, de l'aluminium, du cérium, d'un métal noble et d'un métal du groupe secondaire VIII, et la surface spécifique du cuivre est au maximum de 10 m²/g.

8. Procédé suivant la revendication 7, **caractérisé en ce que** la matière du support comporte un mélange de TiO₂ et d'Al₂O₃ ou un mélange de TiO₂ et de ZrO₂ ou un mélange de TiO₂ et d'Al₂O₃ et de ZrO₂.

9. Procédé suivant la revendication 7 ou 8, **caractérisé en ce que** la matière du catalyseur est, avec addition de poudre de cuivre métallique, déformée en comprimés.

10. Procédé suivant l'une des revendications 1 à 9, **caractérisé en ce que** l'hydrogénation catalytique est effectuée sous la forme d'une réaction en lit fixe suivant un mode de fonctionnement à ruissellement ou un mode de fonctionnement en fond de cuve.

11. Procédé suivant l'une des revendications 1 à 10, **caractérisé en ce que** le procédé est effectué dans un mode de fonctionnement en circulation.
